Europäisches Patentamt

European Patent Office

Office européen des brevets

(19)

(11) Publication number: **0 251 440**
**A2**

# (12) EUROPEAN PATENT APPLICATION

(21) Application number: 87303629.7

(22) Date of filing: 24.04.87

(51) Int. Cl.⁴: **C12N 15/00** , **C12P 21/00** , **G01N 33/577** , **A61K 39/395**

The applicant has filed a statement in accordance with Rule 28 (4) EPC (issue of a sample only to an expert). Accession number(s) of the deposit(s):ECACC 86041803,ECACC 87021301.

(30) Priority: **25.04.86 GB 8610106**

(43) Date of publication of application:
**07.01.88 Bulletin 88/01**

(84) Designated Contracting States:
**DE GB NL SE**

(71) Applicant: **Central Blood Laboratories Authority**
**"The Crest", Dagger Lane, Elstree,**
**Boreham Wood, Hertfordshire, WD6 3AU(GB)**

(72) Inventor: **Hughes-Jones, Nevin Campbell**
**65 Orchard Road Melbourn**
**Royston Hertfordshire SG8 6BB(GB)**
Inventor: **Thompson, Keith Michael**
**140 Malvern Road**
**Cherry Hinton Cambridge CB1 4LH(GB)**
Inventor: **Melamed, Mark David**
**5 Dalmeny Road**
**London N7 0HG(GB)**

(74) Representative: **Holmes, Michael John et al**
**Frank B. Dehn & Co. Imperial House 15-19**
**Kingsway**
**London WC2B 6UZ(GB)**

(54) **Human anti-Rhesus D producing heterohybridomas.**

(57) The present invention provides an anti-Rhesus D (RhD) monoclonal antibody producing heterohybridoma formed by fusion of non-Ig secreting mouse myeloma cells with an anti-RhD Ig producing population of Epstein Barr virus (EBV)-transformed human B lymphocytes, which heterohybridoma when cultured at 37°C in hybridoma culture medium (RPMI-I640 supplemented with I0% (v/v) fetal calf serum or a medium which sustains equal growth under the same conditions) at about $I \times I0^6$ cells/ml produces more than 20 µg anti-RhD monoclonal antibody/ml/24hrs. Two heterohybridomas which produce IgM anti-RhD monoclonal antibodies, designated MAD-2 and FOM-I, have been deposited at the European Collection of Animal Cell Cultures, UK under accession nos. 86041803 and 87021301.

EP 0 251 440 A2

# Human anti-Rhesus D producing heterohybridomas

The present invention relates to new mouse-human heterohybridomas which secrete human monoclonal antibodies to the human erythrocyte Rhesus D antigen (hereinafter referred to as the RhD antigen), e.g. human anti-RhD monoclonal antibodies of the IgM or IgG class suitable for blood-typing or passive immunisation of RhD$^-$ mothers to prevent haemolytic disease of the newborn (HDNB).

HDNB arises in newborn RhD$^+$ infants of RhD$^-$ mothers previously sensitized to RhD antigen as a result of anti-RhD IgG antibodies crossing the placenta during pregnancy and causing fetal red cell destruction. Sensitization of the RhD$^-$ mother to RhD antigen may have occurred at the birth of an earlier RhD$^+$ child due to some fetal red blood cells entering the maternal circulation and being recognised by the maternal immune system. To reduce the incidence of HDNB, it is routine practice in the United Kingdom and many other countries to give anti-RhD antibodies to RhD$^-$ mothers immediately after the birth of an RhD$^+$ infant so that any fetal RhD$^+$ red cells which have entered the maternal circulation are rapidly removed (Mollison, P.L. (1983) Blood Transfusion in Clinical Medicine, 7th edn, Blackwell Scientific, Oxford).

At the present time, anti-RhD antibodies are largely obtained directly from donors immunised during pregnancy or following a mismatched blood transfusion, or by the deliberate immunisation of volunteers. In order to obtain adequate plasma anti-RhD concentrations, many donors require boosting injections of red cells and this procedure, despite of rigorous monitoring, carries the risks common to receiving any transfusion of red cells, e.g. risk of transmission of hepatitis viruses and HIV. Moreover, agglutinating IgM anti-RhD, which is particularly valuable in Rh typing, is very difficult to obtain as it is rarely produced in high titre by immunised donors. Hence, there is much interest in means of producing anti-RhD antibodies in vitro.

It has previously been shown (Boylston et al. Scand. J. Immunol. 12, 355 (1980) and Melamed et al, Eur. J. Immunol. 15, 742 (1985)) that human B lymphocytes from anti-RhD donors can produce anti-RhD in tissue culture after infection with Epstein-Barr virus (EBV), but unfortunately these lymphoblastoid cells usually cease production of anti-RhD after a few months. In order to obtain a more stable human immunoglobulin-secreting cell line, various workers have produced fused hybridomas. For example, in 1973 Schwaber and Cohen reported the first successful fusion between a mouse myeloma line (TEPC-15) and human peripheral blood B lymphocytes (Schwaber & Cohen, Nature 244, 444 (1973)). The resultant heterohybridoma secreted both mouse and human Ig, whereas selective synthesis of human Ig is required for successful production of the required human antibody.

Other fusions have been reported using B cells from spleens (Nowinski et al, Science 210, 537 (1980)), lymph nodes (Schlom et al, Proc. Nat. Acad. Sci. 77, 6841 (1980)) or peripheral blood (Croce et al., Proc. Nat. Acad, Sci. 76, 3416 (1979) and Lane et al, J. Exp. Med. 155, 333 (1982)). For these fusions, the mouse myeloma line NSI or SPI was employed. Successful fusion between EBV-infected human peripheral blood B cells and mouse myeloma cells of the non-Ig secreting cell line X63-Ag8.653 to give heterohybridomas which secrete an IgM anti-tetanus monoclonal antibody has also been disclosed (Kozbor et al, (1982) Hybridoma 1, 323-328). All the heterohybridomas thus obtained by Kozbor et al were found to produce human IgM with kappa light chains. In further studies with four of these heterohybridomas, it was found that when they were seeded into wells containing RPMI-1640 medium supplemented with 10% fetal calf serum (FCS) at $2 \times 10^4$ cells/well/2ml the production of specific anti-tetanus antibody after 9-10 days did not exceed 2 µg/ml. There has further been disclosure in published PCT application 85/02413 of fusion of EBV, transformed human peripheral blood B cells with a myeloma which is itself a heterohybridoma formed between a human myeloma cell and a mouse myeloma cell of the cell line X63-Ag8.653 so as to obtain stable cell lines secreting anti-RhD monoclonal antibodies of the IgM or IgG class. However, it was not disclosed whether the resulting heterohybridomas were capable of producing adequate titres of anti-RhD monoclonal antibody, for example more than 20 µg/ml/24hrs when cultured at about $1 \times 10^6$ cells/m.

We have now found it possible to obtain mouse-human heterohybridomas which when cultured under appropriate conditions provide a high titre of human anti-RhD monoclonal antibody in the absence of mouse Ig by fusing non-Ig secreting mouse myeloma cells directly with EBV-transformed human B lymphocytes derived from RhD hyperimmunised donors. We have found that such human B lymphocytes are compatible with mouse myeloma cells without preliminary fusion with human myeloma cells as in PCT Application 85/02413.

According to one aspect of the present invention, we provide an anti-RhD monoclonal antibody producing heterohybridoma formed by fusion of non-Ig secreting mouse myeloma cells, preferably mouse X63-Ag8.653 myeloma cells, with an anti-RhD Ig producing population of EBV-transformed human B lymphocytes, which heterohybridoma when cultured at 37°C in hybridoma culture medium as hereinafter defined at about $1 \times 10^6$ cells/ml produces more than 20 $\mu$g anti-RhD monoclonal antibody/ml/24hrs. The anti-RhD monoclonal antibody will generally be of the IgM or IgG class.

The term "hybridoma culture medium" as used herein shall be understood to refer to a medium comprising RPMI-1640 medium supplemented with 10% (v/v) FCS or a medium which sustains equal growth under the same conditions.

It is known that only about 1 in $10^3$ or $10^4$ of peripheral blood B lymphocytes in donors hyperimmunised with RhD antigen are anti-RhD secretors (Elson and Bradley, Lancet 1, 789 (1970)). EBV transformation of B lymphocytes isolated from RhD$^-$ donors naturally and/or deliberately immunised with RhD antigen facilitates selection for vigorous growth and high anti-RhD titres in vitro prior to carrying out fusion with the chosen myeloma cells. Moreover, EBV transformed B lymphocytes have been shown to have an improved fusion frequency with mouse X63-Ag8.653 cells compared to non-EBV transformed B lymphocytes under identical condtions. Thus, it has been found that when equal numbers of non-EBV transformed human peripheral blood B lymphocytes and mouse X63-Ag8.653 cells are subjected to conventional fusion conditions, the mean fusion frequency is about $8.4 \times 10^{-6}$. Substitution of the non-EBV transformed B lymphocytes by EBV transformed B lymphocytes results in increase of the mean fusion frequency to about $1.0 \times 10^{-4}$.

For preparation of heterohybridomas according to the present invention, the population of EBV-transformed B lymphocytes chosen for fusion with the mouse myeloma cells desirably produces more than 200 ng of anti-RhD/$10^6$ cells/24hrs, most preferably more than 250 ng of anti-RhD/$10^6$ cells/24hrs when cultured at 37°C in lymphoblastoid cell culture medium as hereinafter defined.

The term "lymphoblastoid cell culture medium" as used herein shall be understood to refer to RPMI-1640 medium supplemented with 10% (v/v) FCS, benzylpencillin K (100 $\mu$g/ml), streptomycin sulphate (100 $\mu$g/ml), amphotericin-B (2.5 $\mu$g/ml), 2-mercaptoethanol (50 $\mu$M) and L-glutamine (2 mM) or an equivalent medium capable of sustaining equal growth of EBV-transformed B lymphocytes under the same conditions.

In order to obtain a preferred population of EBV-transformed B lymphocytes for fusion as defined above, it has been found preferable for the starting population of B lymphocytes to be taken from blood of an RhD hyperimmunised donor at about 13 to 57 days, most preferably about 13 to 28 days, after secondary immunisation. A method of preparing anti-RhD positive, EBV-transformed human B lymphocytes for fusion with an immortalised cell line wherein the starting B lymphocytes are obtained from the blood of an RhD hyperimmunised donor within a time limit as given above constitutes a further aspect of the present invention.

Harvesting of B lymphocytes from blood of a chosen human donor, for example by first isolating the "buffy coat fraction" (white cells), separating the mononuclear cells and removal of T-cells and contaminating platelets, is suitably followed by transformation of isolated B cells with EBV and subsequently one or more growth and selection steps, selection of colonies being on the basis of vigorous growth and high anti-RhD titre. If desired, colonies of isolated B lymphocytes containing cells expressing surface anti-RhD Ig may be selected prior to EBV transformation by rosetting of anti-RhD producing B lymphocytes using papain treated RhD$^+$ red cells. Alternatively, initial colonies of EBV treated B cells may be tested for the presence of B cells expressing surface anti-RhD Ig by the same technique. Quantitation of the anti-RhD titre in the case of colonies positive for this type of immunoglobulin may be carried out in conventional manner. EBV transformation of isolated B cells may be conveniently achieved by contacting the cells with culture supernatant from overgrown cultures of the B95.8 marmoset cell line as described, for example, by Melamed et al. in Eur. J. Immunol (1985) 15, 742-746.

Two hybridoma cell lines according to the present invention, which were obtained by fusing cells of the mouse myeloma cell line X63-Ag8.653 with two different populations of EBV-transformed human B lymphocytes having a high anti-RhD titre derived from peripheral blood of RhD hyperimmunised donors 7 weeks and 13 days after secondary immunisation respectively, have been deposited at the European Collection of Animal Cell Cultures (ECACC), Porton Down, UK. These heterohybridomas, designated respectively MAD-2 and FOM-1, are both capable of producing an anti-RhD monoclonal antibody of the IgM class with lambda light chains. MAD-2 was deposited at the ECACC, UK under accession No. 86041803 on 18th April 1986. FOM-1 was deposited at the ECACC on 13th February 1987 under accession number 87021301.

Further studies on the IgM anti-RhD antibody produced by the cell line MAD-2 have shown that it is a highly suitable antibody for use in Rh-typing in blood transfusion practice. In a potency test comparing MAD-2 cultures (about $1 \times 10^6$ cells/ml of hybridoma culture medium at 37°C for 24hrs) with the U.S. Office of Biologies, Research and Review IgM anti-D standard preparation, the culture supernatants were found to be about 32 times more potent than the reference standard and can thus be diluted for routine use. As shown in Table I below, the MAD-2 IgM has been found to agglutinate all RhD-positive red cells tested and most $D^u$ red cells, but does not react with rare $D^B$ cells (incidence 1 in a 1000). This does not matter for blood transfusion recipients who would be classed as Rh-negative and thus receive Rh-negative blood. Donors classed as Rh negative with this IgM anti-RhD would require to be screened, e.g. with a polyclonal IgG anti-RhD, in order to determine the presence of the $D^u$ and $D^B$ antigens.

## TABLE 1

The results of agglutination tests with the MAD-2 IgM using red cells of differing genotypes.

| Cell genotype | Number of Donors | Agglutination |
|---|---|---|
| $OR_1r$ | | + |
| $OR_2r$ | | + |
| $OR_0r$ | | + |
| $Or'r$ | | − |
| $Or''r$ | | − |
| $A_1rr$ | | − |
| $Orr$ | | − |
| $D^u$ | 5 | $4/5*$ |
| | | $5/5^x$ |
| $D^B$ | 3 | $0/3$ |
| IgG-coated$^{\neq}$ | | − |
| C3-C4-coated$^{\neq}$ | | − |

+      Agglutination present

−      No agglutination

*      4 out of 5 positive after 1 minute incubation

x      After 30 mins incubation

≠      Rh-negative cells strongly coated with IgG anti-c or

         Rh-negative cells coated with the complement components C3 and C4. These cells were included as controls to show that the IgM anti-RhD did not cause non-specific agglutination of cells coated with "non-anti-RhD" IgG.

The new MAD-2 cell line grows well in large scale bulk culture and produces good yields of antibody. A production rate of approximately 2 to 3 litres per week provides sufficient IgM anti-RhD for blood typing in a population of 50 million people with the existing transfusion practices and approximately 20 litres/week will supply sufficient anti-RhD for passive immunisation. Culture supernatants can be obtained after 24hr culture at about 1 × 10⁶ cells/ml with IgM contents of over 20 μg/ml, generally over 25 μg/ml.

FOM-I and other cell lines produced by the similar techniques according to the present invention also have good growth and antibody production characteristics. The IgM secreted by the cell line FOM-I, like the MAD-2 IgM, has been shown to be very specific for the D-component of the Rhesus system and to be very effective at inducing a diagnostic agglutination reaction with RhD-positive cells.

It should be understood that the present invention in addition to encompassing MAD-2 and FOM-I also encompasses IgM anti-RhD producing mutants thereof.

According to a further aspect of the present invention, we provide an unconcentrated supernatant from a cell culture containing over 20μg of human IgM anti-RhD/ml. Such a supernatant may be advantageously directly used for Rh-typing after appropriate dilution.

According to still further aspects of the present invention, we provide a method of Rh-typing of red blood cells using a monoclonal anti-RhD immunoglobulin produced by a heterohybridoma according to the present invention, preferably a monoclonal IgM anti-RhD according to the present invention such as the IgM of MAD-2 or FOM-I, and use of such an antibody for passive immunisation of an RhD⁻ mother after the birth of an RhD⁺ child to prevent sensitization of the mother to RhD antigen. A sterile solution of an antibody according to the present invention suitable for human injection may be formulated in any physiologically acceptable aqueous medium, for example isotonic phosphate-buffered saline or serum. The antibody may if desired be supplied in a freeze-dried formulation ready for dilution prior to use.

The following Examples illustrate the invention further.

## Example I

## Establishment of the IgM anti-RhD secreting heterohybridoma cell line MAD-2.

### (i) Isolation of peripheral blood B cells

The "buffy coat fraction" (white cells) was obtained from blood of an Rh-negative donor who had been initially immunised with six injections of Rh-positive cells (R₂R₂) at the Cambridge Blood Transfusion Centre, Cambridge, UK 10 years previously. The most recent boosting injection was 7 weeks prior to bleeding.

Mononuclear cells were isolated on Ficoll-Hypaque (Pharmacia, Uppsala, Sweden) and contaminating platelets were removed by a second centrifugation with 50% isotonic Percoll solution (10 min at 400g). T cells were then removed using 2-aminoethylisothiouronium bromide-treated sheep red blood cells (Madsen et al. (1980) J. Immunol. Methods 33, 323).

### (ii) EBV transformation of isolated B cells

The marmoset cell line B95.8 (Miller and Lipman (1973) Proc. Natl. Acad. Sci. USA 70,190) was grown in standard tissue culture medium, allowed to become confluent and then incubated for a further week before use. The spent supernatant was harvested, filtered through a 0.8 μm membrane and used to infect B cells at 10⁶ cells/ml for 1.5 to 2 hrs at 37°C (Melamed et al. (1985) Eur. J. Immunol 15, 742-746).

### (iii) Establishment of the lymphoblastoid cell line

The EBV-transformed B cells were grown in 192 flat-bottomed wells (200 μl) at an initial density of 5 × 10⁵/ml (culture medium: RPMI-1640 medium supplemented with 10% (v/v) FCS, benzylpenicillin K(100 μml), streptomycin sulphate (100 μg/ml), amphotericin-B (2.5 μg/ml), 2-mercaptoethanol (50 μM) and L-glutamine (2mM)).

Detection and assay of IgM and IgG anti-RhD obtained from tissue culture wells were carried out by agglutination in microtitre plates using native and papain-treated red cells respectively, at a final concentration of 0.5% cells (Melamed et al (1985) Eur. J. Immunol. 15, 742-746). The sensitivity of the assay for both antibody classes is approximately 1ng/ml.

Growth was established in all wells and anti-RhD was present in 71% of wells 2 weeks later. Two of the wells were selected for their vigorous growth and high anti-RhD titres (greater than 2000) and grown on to reach 80 cm³ flasks. One of the wells was producing IgG anti-RhD and the other was producing IgM anti-RhD. Two months later, the IgG anti-RhD production rate was found to have declined (titre 8I), but production of IgM anti-RhD was stable (titre approximately 5000). The supernatants of both cultures contained $\alpha$, $\gamma$ and $\mu$ heavy chains and kappa and lambda light chains.

(iv) Fusion

In an intial experiment designed to determine the suitability of X63-Ag8.653 as a fusion partner, the fusion efficiency was found to be approximately I in I × I0⁴.

The mouse myeloma X63-Ag8.653 was grown in RPMI-I640 supplemented with I0% (v/v) FCS, 2 mM glutamine, in an atmosphere of 5% $CO_2$ in air, at 37°C. Under these conditions, the cell line has a doubling time of I8-20hrs, and can be maintained in log phase growth by daily subculturing. Prior to fusion, the viability of the cells (determined by nigrosine dye exclusion) exceeded 95%. Periodically, 8-azaguanine was included in the culture medium at 20 μg/ml to eliminate variants that would survive in hypoxanthine-aminopterinthymidine (HAT) medium.

Cells of the selected IgM anti-RhD producing lymphoblastoid cell culture (I × I0⁷) and myeloma cells (I × I0⁷) were added together in a 25 ml plastic container, diluted with phosphate-buffered saline (NaCl I50 mM; PO₄ I0 mM) and spun to a common pellet for I0 min at 200g. After loosening the pellet, Iml of 45% polyethylene glycol 4000 (Merck, Darmstadt, FRG) and 5% dimethylsulphoxide in phosphate-buffered saline were added dropwise over a period of I min with constant agitation. Agitation was continued in a water bath at 37°C for 90 seconds, after which the cells were slowly diluted with saline at room temperature. The cells were centrifuged for 5 min at 200g, then gently resuspended in complete RPMI-I640, and distributed in 96-well trays preseeded with mouse peritoneal cells (2 × I0⁴ per well), such that each well received I × I0⁵ myeloma cells in I00 μl of medium. After 24 hrs incubation, I00 μl of medium containing twice-concentrated HAT and 2 μM ouabain was added. HAT prevents the outgrowth of unfused mouse myeloma cells and ouabain kills unfused lymphoblastoid cells.

Wells were examined after 7 days when feeding was started by replacing half the medium with fresh medium containing HAT and I μM ouabain. Feeding was repeated every 2 or 3 days. Wells positive for growth were screened for immunoglobulin production when cells covered half the bottom of the well. Hybrids of interest were cloned directly from the 96-well trays. HAT/ouabain selection was maintained for 3 weeks after fusion, and the cells passaged through HT-containing medium before culturing in standard tissue culture medium.

(v) Cloning of hybrids

All wells from the fusion showed vigorous growth. No growth was seen in any well from two mock fusions in which the polyethylene glycol step was omitted. Four weeks after fusion, 30 out of 96 wells were producing anti-RhD titres greater than I00. One well was selected for good growth and anti-RhD titre. Hybrids were transferred singly from the culture in log phase growth by use of a micromanipulator and microscope to individual wells of a 96-well tray preseeded with mouse peritoneal feeder cells. In order to ensure that only single cells were transferred, cells were only picked up when alone in the field of a high power lens, and expelled into an intermediate vessel for examination before being moved to the final well. The cloned cells were recloned after 4 weeks.

After the first cloning, I0 out of I3 of the subclones were positive for anti-RhD titre. After the second cloning, 33 out of 40 subclones were positive for anti-RhD production.

Supernants were screened for the production of human immunoglobulin by an enzyme-linked immunoassay. EIA trays (Dynatech, Bilingshurst, Sussex) were coated with goat anti-polyvalent human immunoglobulin antibodies (Sigma, Poole, Dorset). Culture supernatants were then incubated in the coated trays. Trays were washed with phosphate-buffered saline containing 0.I% Tween 20, and then incubated with anti-human heavy chain or light chain specific antibodies conjugated to alkaline phosphatase (Sigma). After a final wash, trays were incubated with p-nitrophenyl phosphate and colour changes monitored on a 'Titertek Multiskan' EIA reader.

Following the second cloning, the cloned heterohybridoma exhibiing the highest human IgM anti-RhD production was designated MAD-2.

Example 2

Large scale culturing of the MAD-2 cell line

Cells of the MAD-2 cell line (ECACC No. 86041803) were grown in a 20 litre spinner culture using as medium RPMl-l640 at 37°C. The percentage of cells producing IgM anti-RhD initially was 90% (as determined by recloning) and this level was found to be unchanged after several months. IgM anti-RhD concentrations after 24 hours of culture at $1 \times 10^6$ cells/ml were found to be 25 to 50 μg/ml. [Quantitation of the anti-RhD present in culture supernants was carried out by an inhibition immunoassay. $^{125}$I-labelled anti-RhD and $R_2R_2$ red cells were incubated in the presence and absence of culture supernatant and the reaction mixture brought into equilibrium. The amount of $^{125}$I-labelled anti-RhD bound to the red cells was then determined and the concentration of anti-RhD in the culture supernatant was calculated by comparison with a standardization curve using the anti-D International Standard (68/419)].

The IgM reacted with all RhD-positive red cells and with some $D^u$ cells, but not with Rh-negative or $D^B$ cells. When added at a final concentration of 5 μg/ml to a 1% suspension of RhD positive cells, it brought about complete agglutination in about 30 seconds. The antibody has been found to be stable at 4°C over an 8 month period under aqueous solution.

Example 3

Establishment of anti-RhD secreting heterohybridomas using B cells taken from RhD hyperimmunised donors at different times following secondary immunisation

(i) Blood samples

Blood samples were provided by the North London Regional or Cambridge Regional Blood Transfusion centres in the UK from members of their panels of volunteer anti-RhD plasma donors.

Over a 3 year period, 55 "buffy coat fractions" were processed from routine anti-RhD plasma donations of RhD hyperimmunised donors with high levels of circulating anti-RhD antibody. These plasma donations were selected without reference to the time after the last booster immunisation.

20ml samples of anti-coagulated blood were also taken weekly from 5 RhD hyperimmunised individuals for 6-8 weeks after booster immunisation. Immunisation was done by intravenous injection of 5mls of a 10% suspension in isotonic saline of washed RhD$^+$ red cells selected with stringent safety precautions (Gibson (1973) Vox. Sang. 24, 425).

Of the 5 donors, 4 yielded a peak in IgM anti-RhD titre at 7 to 10 days after the booster immunisation, but there were no significant changes in IgG anti-RhD levels. 4 donors examined for peripheral lymphocytes expressing membrane-bound anti-RhD all showed a marked peak between 7 and 16 days after boost. A second rise in the number of "rosettes" after about 3 weeks was due to the appearance of aggregates or clusters of loosely attached red cells presumably held together by secreted antibody. These were quite distinct in appearance from the strictly monolayer rosettes characteristic of membrane Ig seen in the first week.

(ii) Isolation of peripheral blood B cells

From the "buffy coat fractions" derived from routine anti-RhD plasma donations, blood platelets were removed by centrifugation in 50% isotonic Percoll (10 minutes at 400g). T-cells were removed by rosetting with sheep red blood cells in 4% dextran (Brown et al (1975) Clin. Exp. Immunol. 20, 505).

Cells from the 20 ml blood samples were collected at a Ficoll-Hypaque interface and washed twice with sterile saline, collecting the cells by centrifugation at 200g for 5 minutes.

To separate B cells expressing anti-RhD surface Ig from mononuclear cells collected at a Ficoll-Hypaque interface, the following "rosetting" technique was used. Collected mononuclear cells were suspended in medium as used in Example l(iii) (0.2 ml at $10^6$ cells/ml), mixed with well-washed papain-teated RhD-positive red cells (ratio 1:10) and the mixture centrifuged at 200g for 5 minutes. After 30 minutes incubation at room temperature, the supernatant was removed and the pellet gently resuspended in medium containing 2.5 μg/ml fluorescein diacetate (Ramasamy and Munro (1974) Immunol. 26, 563). The number of rosettes in $10^5$ cells were counted using a bright light field microscope. Rhesus negative cells were used as controls and never gave any appreciable counts.

### (iii) EBV transformation of isolated B cells

Virus treatment of isolated B cells in the absence of T-cells was carried out by the method of Melamed et al. as in Example I(ii).

B cells which were not T-cell depleted prior to EBV transformation were exposed to the virus at $4 \times 10^6$ cells/ml and placed out at $2 \times 10^5$ cells/well (0.2ml) in culture medium containing 1:100 phytohaemagglutinin (Moss et al. (1979) Int. J.Cancer, 23, 618).

### (iv) Establishment of lymphoblastoid cell lines

(a) In the case of the EBV-treated B cells derived from the blood samples collected at random times, the same procedure was used as in Example I(iii). Only 5 of the initial populations of EBV-treated B cells yielded anti-RhD lines which could be expanded to the point where successful fusions would have been possible.

(b) The EBV-treated B cells derived from the blood samples taken weekly for about 6-8 weeks after booster immunisation from 5 RhD hyperimmunised donors were distributed in 96 or 192 flat bottomed microplate wells at an initial density of $1 \times 10^6$ cells/ml (medium as in Example I(iii)). The anti-RhD titres of the wells were followed at approximately weekly intervals after transformation. A transient specific antibody response was observed in a great many of the wells over the first 2 weeks. This phase of the response was difficult to quantitate, but titres (determined as in Example I(iii)) generally remained below 100. After about 3 weeks most of this initial response had declined and most of the wells had increased in cell number. Those cultures with the highest titres were transferred up to 24-well plates. It was in the next phase of the response that significant differences were obseved in samples from the same donor but collected at different times after boost. In the case of those samples collected in the period about 2 to 4 weeks after boost, some of the wells continued to increase in titre and cell number and when transferred up to 24-well plates had titres of 250-6000. In samples collected outside this time window, continuous increase in titre was only rarely observed. At 3-4 weeks after EBV transformation, cells from the 3 wells for each time point for each donor with the highest titre were selected for fusion.

### (v) Fusion and cloning

The selected populations of EBV-treated cells were fused with cells of the mouse myeloma cell line X63-Ag8.653 as in Example I(iv). Wells showing consistently increasing anti-RhD titres were chosen for cloning about 3 to 4 weeks after fusion, and cloning and expansion of selected clones repeated until about 90% of the daughter cells produced anti-RhD.

Successful establishment of hybridomas producing anti-RhD mainly occurred with lymphoblastoid cell line (LCL) cultures in which the anti-RhD titre was greater than 250, representing a concentration of anti-RhD of approximately 250 ng/ml. The total Ig in a corresponding well is usually about 25 μg/ml and thus at least 1% of the lymphoblastoid cells were producing anti-RhD in those above-mentioned cultures which yielded specific hybridomas. From the results, it can be concluded that it was the productive LCL cultures which derived mainly from B cells collected about 2 to 4 weeks post boost which predominantly yielded the specific heterohybridomas.

Table II below shows for each of the 11 anti-RhD secreting heterohybridoma cell lines established, the class and light chain type of the anti-RhD monoclonal antibody and the number of days after secondary immunisation when the B cells were collected from the RhD hyperimmunised donor.

8

## TABLE II

| Heterohybridoma | Class of* antibody | Light chain type of antibody | Protein A$^\tau$ binding | Days post boost |
|---|---|---|---|---|
| 1 (FOM-1) | IgM | L | − | 13 |
| 2 | IgG1 | L | + | 13 |
| 3 | IgG3 | K | − | 13 |
| 4 | IgG1 | K | + | 21 |
| 5 | IgG3 | K | − | 21 |
| 6 | IgM | L | + | 57 |
| 7 | IgM | K | + | 13 |
| 8 | IgM | L | − | 13 |
| 9 | IgM | L | + | 20 |
| 10 | IgM | L | + | 27 |
| 11 | IgG1 | L | + | 27 |

*      IgG and IgM antibodies were distinguished on the basis of their reactivity with native and papain-modified red cells. IgG subclasses where identified by an indirect agglutination assay with subclass specific antisera (Dutch Red Cross, Amsterdam)

$\tau$      Protein A binding by each monoclonal antibody was determined by passing the antibody in PBS slowly through a column of Protein A agarose (Sigma Chemical Company). Bound antibody was eluted with citrate buffer at pH 4.0 or, if necessary, at pH 3.5.

All the above-noted heterohybridomas produce anti-RhD monoclonal antibody at a rate of over 20 μg/ml/24hrs when cultured at 37°C in hybridoma culture medium at about I × 10⁶ cells/ml.

Example 4

Use of the MAD-2 IgM for Rh-typing of red blood cells

### (i) Preparation of the blood grouping reagent

A culture supernatant from a culture of the MAD-2 cell line containing 25-50 ug/ml anti RhD monoclonal antibody was diluted as appropriate in 0.05M phosphate buffer at pH7.2, containing 0.IM NaCl, 50g/l bovine albumin, 2.4mM sodium EDTA and Ig/l sodium azide. The reagent was further supplemented with phenol red to detect any minor changes in pH and sterilized by filtration prior to storage at 2-8°C. Each batch of blood grouping reagent was assessed for potency against a recognised anti-RhD standard preparation.

### (ii) Test procedures

A. Tube Technique: centrifugation
 I. Prepare a 3% suspension of washed red cells in 9g/l NaCl.
 2. Place two volumes of Blood Grouping Reagent in a I0 $\times$ 75mm or I2 $\times$ 75mm tube.
 3. Add one volume of cell suspension.
 4. Mix well, leave for I minute, and centrifuge the tube at a suitable rcf and time, e.g. 500 rcf for 60 seconds.
 5. Gently resuspend the cell button and observe macroscopically for agglutination over a white background. Read the negatives microscopically.
B. Technique: sedimentation 37°C
 I. Prepare a 3% suspension of washed red cells in 9gl NaCl.
 2. Place one volume of Blood Grouping Reagent in a 6 $\times$ 50mm tube.
 3. Add an equal volume of cell suspension.
 4. Mix well and incubate at 37°C for 60 minutes.
 5. Gently resuspend the cell button and observe macroscopically for agglutination over a white background. Read the negatives microscopically.
C. Tube Technique: sedimentation 20°C
 I. Prepare a 3% suspension of washed red cells in 9gl NaCl.
 2. Place one volume of Blood Grouping Reagent in a 6 $\times$ 50mm tube.
 3. Add an equal volume of cell suspension.
 4. Mix well and incubate at room temperature (limits I6-25°C) for 60 minutes.
 5. Gently resuspend the cell button and observe macroscopically for agglutination over a white background. Read the negatives microscopically.

### Claims

I. An anti-Rhesus D (RhD) monoclonal antibody producing heterohybridoma characterised in that it is formed by fusion of non-Ig secreting mouse myeloma cells with an anti-RhD Ig producing population of Epstein Barr Virus (EBV)-transformed human B lymphocytes, and in that it produces more than 20µg anti-RhD monoclonal antibody/ml/24hrs when cultured at 37°C, at about I $\times$ I0$^6$ cells/ml, in hybridoma culture medium comprising RPMI-I640 medium supplemented with I0% (v/v) fetal calf serum (FCS) or a medium which sustains equal growth under the same conditions.

2. A heterohybridoma as claimed in claim I wherein the myeloma cell fusion partner is a cell of the mouse myeloma cell line X63-Ag8.653.

3. A heterohybridoma as claimed in claim I or claim 2 which produces an anti-RhD monoclonal antibody of the IgM class.

4. A heterohybridoma as claimed in claim I or claim 2 which produces an anti-RhD monoclonal antibody of the IgG class.

5. A heterohybridoma as claimed in any one of claims I to 4 formed by employing for fusion with the mouse myeloma cells a population of EBV-transformed human B lymphocytes capable of producing more than 200 ng anti-RhD/I0$^6$cells/24hrs when cultured at 37°C in lymphoblastoid cell culture medium (RPMI-I640 medium supplemented with I0% (v/v) FCS, benzylpencillin K (I00 µg/ml), streptomycin sulphate (I00 µg/ml), amphotericin-B (2.5 µg/ml), 2-mercaptoethanol (50 µM) and L-glutamine (2 mM) or an equivalent medium capable of sustaining equal growth of EBV-transformed B lymphocytes under the same conditions).

6. A heterohybridoma as claimed in claims 5 formed by employing for fusion with the mouse myeloma cells a population of EBV-transformed human B lymphocytes capable of producing more than 250 ng anti-RhD/$10^6$cells/24hrs when cultured at 37°C in lymphoblastoid cell culture medium as defined in claim 5.

7. A heterohybridoma as claimed in any one of claims 1 to 6 wherein the B lymphocyte fusion partner is derived from human B lymphocytes isolated from blood of an RhD hyperimmunised donor at about 13-57 days after secondary immunisation.

8. A heterohybridoma as claimed in claim 7 wherein the B lymphocyte fusion partner is derived from human B lymphocytes isolated from blood of an RhD hyperimmunised donor at about 13-28 days after secondary immunisation.

9. The IgM anti-RhD producing heterohybridoma deposited at the European Collection of Animal Cell Cultures under accession No. 86041803 and IgM anti-RhD producing mutants thereof.

10. The IgM anti-RhD producing heterohybridoma deposited at the European Collection of Animal Cell Cultures under accession No. 87021301 and IgM anti-RhD producing mutants thereof.

11. An anti-RhD monoclonal antibody characterised in that it is produced by a heterohybridoma as claimed in any one of claims 1 to 10 and RhD antigen-binding fragments thereof.

12. The IgM anti-RhD monoclonal antibody producible by the heterohybridoma of claim 9 and RhD antigen-binding fragments thereof.

13. The IgM anti-RhD monoclonal antibody producible by the heterohybridoma of claim 10 and RhD antigen-binding fragments thereof.

14. An unconcentrated supernatant from a cell culture characterised in that it contains over 20$\mu$g of human anti-RhD per ml.

15. A method of preparing an anti-RhD monoclonal antibody characterised in that it comprises cultivating a heterohybridoma as claimed in any one of claims 1 to 10 under antibody-producing conditions.

16. A method of preparing a heterohybridoma as claimed in claim 5 which comprises the stages of:

(a) isolating B lymphocytes from a human donor positive for anti-RhD Ig;

(b) transforming isolated B lymphocytes thus obtained with EBV and subsequently carrying out one or more growth and selection steps so as to obtain a population of EBV-transformed B lymphocytes for cell fusion capable of producing more than 200 ng anti-RhD/$10^6$cells/24hrs when cultured at 37°C in lymphoblastoid cell culture medium as defined in claim 5;

(c) fusing EBV transformed B lymphocytes of said population with cells of a non-Ig secreting mouse myeloma cell line; and

(d) selecting anti-RhD monoclonal antibody producing heterohybridomas formed in stage (c) which when cultured at 37°C in hybridoma culture medium as defined in claims 1 at about 1 x $10^6$ cells/ml produce more than 20 $\mu$g anti-RhD monoclonal antibody/ml/24hrs.

17. A method as claimed in claim 16 wherein the mouse myeloma cells employed are cells of the mouse myeloma cell line X63-Ag8.653.

18. A method as claimed in claim 16 or claim 17 wherein in stage (a) B lymphocytes are isolated from blood of a human RhD hyperimmunised donor about 13-57 days after secondary immunisation.

19. A method of preparing an anti-RhD Ig producing population of EBV-transformed human B lymphocytes for fusion with an immortalised cell line, characterised in that the starting B lymphocytes are obtained from the blood of a human RhD hyperimmunised donor about 13-57 days after secondary immunisation.

20. A method of Rh-typing red blood cells, characterised in that an anti-RhD monoclonal antibody as claimed in any one of claims 11 to 13 is employed.

21. An anti-RhD monoclonal antibody as claimed in any one of claims 11 to 13 for use in Rh-typing of red blood cells.

22. A sterile solution of one or more anti-RhD monoclonal antibodies as claimed in any one of claims 11 to 13 suitable for human injection.

23. An anti-RhD monoclonal antibody as claimed in any one of claims 11 to 13 for passive immunisation of an RhD$^-$ mother after the birth of an RhD$^+$ child to prevent sensitization of the mother to RhD antigen.